# EUROPÄISCHE PATENTSCHRIFT

(11) **EP 0 915 852 B1**
(45) Veröffentlichungstag und Bekanntmachung des Hinweises auf die Patenterteilung: **29.12.2004**
(21) Anmeldenummer: 97933662.5
(22) Anmeldetag: 11.07.1997
(51) Int. Cl.: C07D 231/16, A01N 43/56

(54) **1,3-DIMETHYL-5-FLUOR-PYRAZOL-4-CARBOXAMIDE DERIVATIVE, DEREN HERSTELLUNG UND DEREN VERWENDUNG ALS MIKROBIZIDE**
1,3-DIMETHYL-5-FLUORO-PYRAZOLE-4-CARBOXAMIDE DERIVATIVES, THEIR PREPARATION AND THEIR USE AS MICROBICIDES
DERIVES DE 1,3-DIMETHYLE-5-FLUOR-PYRAZOL-4-CARBOXAMIDE, LEUR PREPARATION ET LEUR UTILISATION COMME MICROBICIDES

(30) Priorität: 24.07.1996 DE 19629826
(43) Veröffentlichungstag der Anmeldung: 19.05.1999
(73) Patentinhaber: Bayer CropScience AG, 40789 Monheim (DE)
(72) Erfinder: ELBE, Hans-Ludwig, D-42329 Wuppertal (DE); BIELEFELDT, Dietmar, D-40883 Ratingen (DE); TIEMANN, Ralf, D-51375 Leverkusen (DE); DUTZMANN, Stefan, D-40764 Langenfeld (DE); STENZEL, Klaus, D-40595 Düsseldorf (DE); KUGLER, Martin, D-42799 Leichlingen (DE); WACHTLER, Peter, D-51061 Köln (DE)
(86) Internationale Anmeldenummer: PCT/EP1997/003692
(87) Internationale Veröffentlichungsnummer: WO 1998/003486

(56) Entgegenhaltungen:
- EP-A- 0 589 313
- WO-A-93/11117
- DE-A- 2 701 091
- CHEMICAL ABSTRACTS, vol. 109, no. 25, 19.Dezember 1988 Columbus, Ohio, US; abstract no. 231009v, NISHIDA S. ET AL.: "Preparation of 5-fluoro-pyrazole-4-carboxamides as agrochemical microbicides" Seite 861; Spalte 2; XP002043582 & JP 63 048 269 A (SUMITOMO CHEMICAL CO., LTD.) 29.Februar 1988

## Beschreibung

Die vorliegende Erfindung betrifft neue 1,3-Dimethyl-5-fluor-pyrazol-4-carboxamide, ein Verfahren zu deren Herstellung und deren Verwendung als Mikrobizide.

Es ist bereits bekannt, daß zahlreiche Pyrazolcarboxamide fungizide Eigenschaften besitzen (vgl. WO 93-11 117 und EP-A 0 589 313). So lassen sich z.B. 1,3-Dimethyl-5-fluor-pyrazol-4-carbonsäure-(2-cycloheptyl)-anilid und 1,3-Dimethylpyrazol-4-carbonsäure-N-(2-sec.-butyl-cyclohexyl)-amid zur Bekämpfung von Pilzen verwenden. Die Wirksamkeit dieser Stoffe ist gut, läßt aber bei niedrigen Aufwandmengen in manchen Fällen zu wünschen übrig.

Es wurden nun neue 1,3-Dimethyl-5-fluor-pyrazol-4-carboxamide der Formel 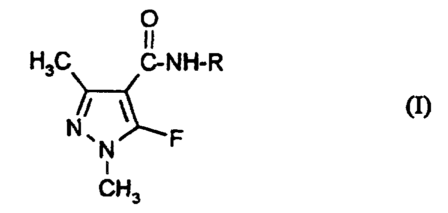 in welcher
- R: für eine Gruppierung der Formel 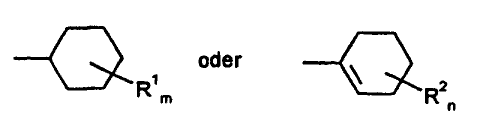 steht, worin
- R¹: für geradkettiges oder verzweigtes Alkyl mit 1 bis 12 Kohlenstoffatomen, Cycloalkyl mit 3 bis 8 Kohlenstoffatomen, Bicycloalkyl mit 7 bis 12 Kohlenstoffatomen, gegebenenfalls einfach bis dreifach, gleichartig oder verschieden durch Halogen und/oder Alkyl mit 1 bis 4 Kohlenstoffatomen substituiertes Phenyl oder für gegebenenfalls einfach bis dreifach, gleichartig oder verschieden durch Halogen und/oder Alkyl mit 1 bis 4 Kohlenstoffatomen substituiertes Phenylalkyl mit 1 bis 4 Kohlenstoffatomen im Alkylteil steht,
- m: für die Zahlen 0, 1, 2 oder 3 steht,
oder
zwei vicinale R¹-Reste gemeinsam für eine Alkylenkette mit 3 oder 4 Kohlenstoffatomen stehen,
- R²: für geradkettiges oder verzweigtes Alkyl mit 1 bis 12 Kohlenstoffatomen, Cycloalkyl mit 3 bis 8 Kohlenstoffatomen, Bicycloalkyl mit 7 bis 12 Kohlenstoffatomen, gegebenenfalls einfach bis dreifach, gleichartig oder verschieden durch Halogen und/oder Alkyl mit 1 bis 4 Kohlenstoffatomen substituiertes Phenyl oder für gegebenenfalls einfach bis dreifach, gleichartig oder verschieden durch Halogen und/oder Alkyl mit 1 bis 4 Kohlenstoffatomen substituiertes Phenylalkyl mit 1 bis 4 Kohlenstoffatomen im Alkylteil steht und
- n: für die Zahlen 0, 1, 2 oder 3 steht,
oder
zwei vicinale R²-Reste gemeinsam für eine Alkylenkette mit 3 oder 4 Kohlenstoffatomen stehen.
gefunden.

Weiterhin wurde gefunden, daß man 1,3-Dimethyl-5-fluor-pyrazol-4-carboxamide der Formel (I) erhält, wenn man 1,3-Dimethyl-5-fluor-pyrazol-4-carbonsäurehalogenide der Formel 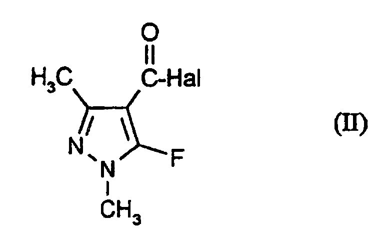 in welcher
- Hal: für Fluor, Chlor oder Brom steht,
mit Aminen der Formel

H₂N-R (III)

in welcher
- R: die oben angegebenen Bedeutungen hat,
gegebenenfalls in Gegenwart eines Säurebindemittels und gegebenenfalls in Gegenwart eines Verdünnungsmittels umsetzt.

Schließlich wurde gefunden, daß die neuen 1,3-Dimethyl-5-fluor-pyrazol-4-carboxamide der Formel (I) sehr gute mikrobizide Eigenschaften besitzen und zur Bekämpfung unerwünschter Mikroorganismen sowohl im Pflanzenschutz als auch im Materialschutz eingesetzt werden können.

Überraschenderweise zeigen die erfindungsgemäßen 1,3-Dimethyl-5-fluor-pyrazol-4-carboxamide der Formel (I) eine wesentlich bessere fungizide Wirksamkeit als das 1,3-Dimethyl-5-fluor-pyrazol-4-carbonsäure-(2-cycloheptyl)-anilid und das 1,3-Dimethyl-pyrazol-4-carbonsäure-N-(2-sec.-butyl-cyclohexyl)-amid, welches konstitutionell ähnliche, vorbekannte Wirkstoffe gleicher Wirkungsrichtung sind.

Die erfindungsgemäßen 1,3-Dimethyl-5-fluor-pyrazol-4-carboxamide sind durch die Formel (I) allgemein definiert.
- R: für eine Gruppierung der Formel 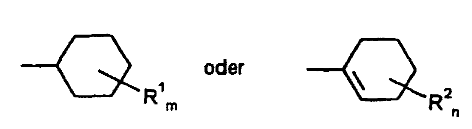 steht, worin
R¹ für Methyl, Ethyl, n-Propyl, Isopropyl, n-Butyl, sek.-Butyl, iso-Butyl, tert.-Butyl, 2-Ethyl-butyl, Octyl, Decyl, Dodecyl, Cyclopropyl, Cyclopentyl, Cyclohexyl, Cycloheptyl, Cyclooctyl, Bicyclo[2,2,1]heptyl, gegebenenfalls einfach oder zweifach durch Fluor, Chlor, Brom, Methyl, Ethyl, n-Propyl, Isopropyl und/oder tert.-Butyl substituiertes Phenyl oder für Phenylalkyl mit 1 bis 4 Kohlenstoffatomen im Alkylteil steht, wobei der Phenylteil einfach oder zweifach durch Fluor, Chlor, Brom, Methyl, Ethyl, n-Propyl, Isopropyl und/oder tert.-Butyl substituiert sein kann,
m für die Zahlen 0, 1, 2 oder 3 steht, wobei R¹ für gleiche oder verschiedene Reste steht, wenn m für 2 oder 3 steht,
oder
zwei vicinale R¹-Reste gemeinsam für eine Alkylenkette mit 3 oder 4 Kohlenstoffatomen stehen,
R² für Methyl, Ethyl, n-propyl, Isopropyl, n-Butyl, sek.-Butyl, iso-Butyl, tert.-Butyl, 2-Ethyl-butyl, Octyl, Decyl, Dodecyl, Cyclopropyl, Cyclopentyl, Cyclohexyl, Cycloheptyl, Cyclooctyl, Bicyclo[2,2,1]heptyl, gegebenenfalls einfach oder zweifach durch Fluor, Chlor, Brom, Methyl, Ethyl, n-Propyl, Isopropyl und/oder tert.-Butyl substituiertes Phenyl oder für Phenylalkyl mit 1 bis 4 Kohlenstoffatomen im Alkylteil steht, wobei der Phenylteil einfach oder zweifach durch Fluor, Chlor, Brom, Methyl, Ethyl, n-Propyl, Isopropyl und/oder tert.-Butyl substituiert sein kann und
n für die Zahlen 0, 1, 2 oder 3 steht, wobei R² für gleiche oder verschiedene Reste steht, wenn n für 2 oder 3 steht,
oder
zwei vicinale R²-Reste gemeinsam für eine Alkylenkette mit 3 oder 4 Kohlenstoffatomen stehen.

Verwendet man 1,3-Dimethyl-5-fluor-pyrazol-4-carbonsäure-fluorid und 2-(2-Ethyl-but-1-yl)-cyclohexylamin als Ausgangsstoffe, so kann der Verlauf des erfindungsgemäßen Verfahrens durch das folgende Formelschema veranschaulicht werden.

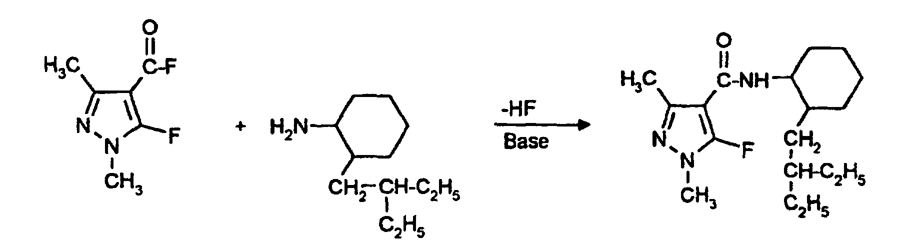

Die bei der Durchführung des erfindungsgemäßen Verfahrens als Ausgangsstoffe benötigten 1,3-Dimethyl-5-fluor-pyrazol-4-carbonsäure-halogenide sind durch die Formel (II) allgemein definiert. Hal steht auch vorzugsweise für Fluor, Chlor oder Brom.

Die 1,3-Dimethyl-5-fluor-pyrazol-4-carbonsäure-halogenide sind teilweise bekannt (vgl. WO 93-11 117). Das 1,3-Dimethyl-5-fluor-pyrazol-4-carbonsäure-fluorid der Formel 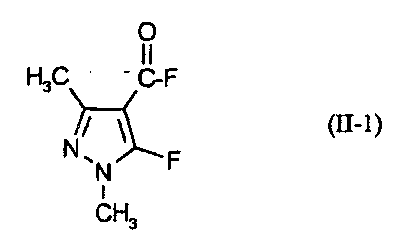 ist bisher noch nicht bekannt. Es läßt sich herstellen, indem man 1,3-Dimethyl-5-chlor-pyrazol-4-carbonsäurechlorid der Formel 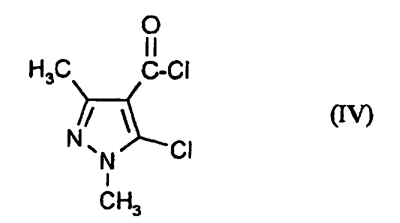 mit Fluoriden gegebenenfalls in Gegenwart eines Verdünnungsmittels umsetzt.

Das bei dem obigen Verfahren als Ausgangsstoff benötigte 1,3-Dimethyl-5-chlorpyrazol-4-carbonsäure-chlorid der Formel (IV) ist bekannt (vgl. JP-A 1990-85 257 und Chem. Abstr. 113, 78 387).

Als Reaktionskomponenten kommen bei dem Verfahren zur Herstellung von 1,3-Dimethyl-5-fluor-pyrazol-4-carbonsäure-fluorid der Formel (II-1) alle üblichen Metallfluoride, Ammoniumfluorid und Phosphoniumfluoride in Frage. Vorzugsweise verwendbar sind Alkalimetallfluoride, wie Kaliumfluorid und Cäsiumfluorid, ferner Ammoniumfluorid oder Triphenylmethylphosphonium-fluorid. Die Fluoride sind bekannt.

Als Verdünnungsmittel kommen bei der Herstellung des 1,3-Dimethyl-5-fluorpyrazol-4-carbonsäure-fluorids der Formel (II-1) nach dem obigen Verfahren alle üblichen polaren, aprotischen organischen Lösungsmittel in Betracht. Vorzugsweise verwendbar sind Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol, ferner Ketone, wie Aceton, Butanon, Methylisobutylketon oder Cyclohexanon, außerdem Nitrile, wie Acetonitril, Propionitril, n- oder i-Butyronitril oder Benzonitril; weiterhin Amide, wie N,N-Dimethylformamid, N,N-Dimethylacetamid, N-Methylformanilid, N-Methylpyrrolidon oder Hexamethylphosphorsäuretriamid; ferner Ester, wie Essigsäuremethylester oder Essigsäureethylester, und auch Sulfoxide, wie Dimethylsulfoxid, oder Sulfone, wie Sulfolan.

Die Reaktionstemperaturen können bei dem obigen Verfahren zur Herstellung des 1,3-Dimethyl-5-fluor-pyrazol-4-carbonsäure-fluorids der Formel (II-1) in einem größeren Bereich variiert werden. Im allgemeinen arbeitet man bei Temperaturen zwischen 100 und 250°C, vorzugsweise zwischen 150 und 200°C.

Bei der Herstellung des 1,3-Dimethyl-5-fluor-pyrazol-4-carbonsäure-fluorids der Formel (II-1)- nach dem obigen Verfahren arbeitet man im allgemeinen unter Atmosphärendruck. Es ist aber auch möglich, unter erhöhtem oder vermindertem Druck zu arbeiten.

Bei der Herstellung des 1,3-Dimethyl-5-fluor-pyrazol-4-carbonsäure-fluorids der Formel (II-1) nach dem obigen Verfahren setzt man auf 1 Mol an 1,3-Dimethyl-5-chlor-pyrazol-4-carbonsäure-chlorid der Formel (IV) im allgemeinen 2 bis 15 Mol, vorzugsweise 2 bis 4 Mol an Fluorid ein. Im einzelnen geht man im allgemeinen so vor, daß man eine Lösung von Fluorid in einem Solvens mit 1,3-Dimethyl-5-chlor-pyrazol-4-carbonsäure-chlorid versetzt und das entstehende Gemisch bis zur Beendigung der Umsetzung auf die gewünschte Temperatur erhitzt. Die anschließende Aufarbeitung erfolgt nach üblichen Methoden. Im allgemeinen verfährt man in der Weise, daß man das Reaktionsgemisch einer fraktionierten Destillation unter vermindertem Druck unterwirft.

Die bei der Durchführung des erfindungsgemäßen Verfahrens als Reaktionskomponenten benötigten Amine sind durch die Formel (III) allgemein definiert. In dieser Formel hat R vorzugsweise diejenigen Bedeutungen, die bereits im Zusammenhang mit der Beschreibung der erfindungsgemäßen Stoffe der Formel (I) für diesen Rest als bevorzugt genannt wurden.

Die Amine der Formel (III) sind bekannt oder lassen sich nach bekannten Verfahren herstellen (vgl. EP-A 0 529 313).

Als Säurebindemittel kommen bei der Durchführung des erfindungsgemäßen Verfahrens alle für derartige Reaktionen üblichen anorganischen und organischen Basen in Betracht. Vorzugsweise verwendbar sind Erdalkali- oder Alkalimetallhydroxide, wie Natriumhydroxid, Calciumhydroxid, Kaliumhydroxid, oder auch Ammoniumhydroxid, Alkalimetallcarbonate, wie Natriumcarbonat, Kaliumcarbonat, Kaliumhydrogencarbonat, Natriumhydrogencarbonat, Alkali- oder Erdalkalimetallacetate wie Natriumacetat, Kaliumacetat, Calciumacetat, sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU). Es ist jedoch auch möglich, ohne zusätzliches Säurebindemittel zu arbeiten, oder die Aminkomponente in einem Überschuß einzusetzen, so daß sie gleichzeitig als Säurebindemittel fungiert.

Als Verdünnungsmittel kommen bei der Durchführung des erfindungsgemäßen Verfahrens alle üblichen inerten, organischen Solventien in Frage. Vorzugsweise verwendbar sind gegebenenfalls halogenierte aliphatische, alicyclische oder aromatische Kohlenwasserstoffe, wie Petrolether, Hexan, Heptan, Cyclohexan, Methylcyclohexan, Benzol, Toluol, Xylol oder Decalin; Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester, wie Essigsäuremethylester oder Essigsäureethylester; Sulfoxide, wie Dimethylsulfoxid, oder Sulfone, wie Sulfolan.

Die Reaktionstemperaturen können bei der Durchführung des erfindungsgemäßen Verfahrens in einem größeren Bereich variiert werden. Im allgemeinen arbeitet man bei Temperaturen zwischen 0°C und 100°C, vorzugsweise zwischen 10°C und 80°C.

Bei der Durchführung des erfindungsgemäßen Verfahrens arbeitet man im allgemeinen unter Atmosphärendruck. Es ist aber auch möglich, unter erhöhtem oder vermindertem Druck zu arbeiten.

Bei der Durchführung des erfindungsgemäßen Verfahrens setzt man auf 1 Mol an 1,3-Dimethyl-5-fluor-pyrazol-4-carbonsäure-halogenid der Formel (II) im allgemeinen 1 Mol oder auch einen Überschuß an Amin der Formel (III) sowie 1 bis 3 Mol an Säurebindemittel ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, daß man das Reaktionsgemisch mit Wasser versetzt, die organische Phase abtrennt und nach dem Trocknen unter vermindertem Druck einengt. Der verbleibende Rückstand kann gegebenenfalls nach üblichen Methoden, wie Chromatographie oder Umkristallisation, von eventuell noch vorhandenen Verunreinigungen befreit werden.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder
Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Krankheiten im Wein-, Obst- und Gemüseanbau, wie beispielsweise gegen Venturia-, Podosphaera-, Phytophtora- und Plasmopara-Arten, einsetzen. Mit gutem Erfolg werden auch Reiskrankheiten, wie beispielsweise Pyricularia-Arten, bekämpft.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
Calciumpolysulfid, Capsimycin, Captafol, Captan, Carbendazim, Carboxin, Carvon, Chinomethionat(Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclafluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilate, Iminoctadinetriacetat, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,
Quinconazol, Quintozen (PCNB),
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofosmethyl, Tolylfluanid, Triadimefon, Triadimenol, Triazbutil, Triazoxid, Trichlamid, Tricyclazol, Tridemorph, Triflumizol, Triforin, Triticonazol,
Uniconazol,
Validamycin A, Vindozolin, Viniconazol,
Zarilamid, Zineb, Ziram sowie
Dagger G,
OK-8705,
OK-8801,
2',6'-Dibrom-2-methyl-4'-trifluormethoxy-4'-trifluor-methyl-1,3-thiazol-5-carboxanilid,
2,6-Dichlor-N-(4-trifluormethylhenzyl)-benzamid,
2-Aminobutan,
2-Phenylphenol(OPP),
8-Hydroxychinolinsulfat,
cis-1-(4-Chlorphenyl)-2-(1H-1,2,4-triazol-1-yl)-cycloheptanol,
(5RS,6RS)-6-Hydroxy-2,2,7,7-tetramethyl-5-(1H-1,2,4-triazol-1-yl)-3-octanon,
α-(2,4-Dichlorphenyl)-β-methoxy-α-methyl-1H-1,2,4-triazol-1-ethanol,
α-(1,1-Dimethylethyl)-β-(2-phenoxyethyl)-1H-1,2,4-triazol-1-ethanol,
1-[1-[2-[(2,4-Dichlorphenyl)-methoxy]-phenyl]-ethenyl]-1H-imidazol,
bis-(1-Methylethyl)-3-methyl-4-[(3-methylbenzoyl)-oxy]-2,5-thiophendicarboxylat,
2,6-Dichlor-N-[[4-(tnnuormethyl)-phenyl]-methyl]-benzamid,
(E)-α-(Methoxyimino)-N-methyl-2-phenoxy-phenylacetamid,
9H-Xanthen-9-carbonsäure-2-[(phenylamino)-carbonyl]-hydrazid,
O-Methyl-S-phenyl-phenylpropylphosphoramidothioate,
N-(5-Chlor-2-methylphenyl)-2-methoxy-N-(2-oxo-3-oxazolidinyl)-acetamid,
1-(2,4-Dichlorphenyl)-2-(1H-1,2,4-triazol-1-yl)-ethanon-O-(phenylmethyl)-oxim,
N-(2,6-Dimethylphenyl)-2-methoxy-N-(tetrahydro-2-oxo-3-thienyl)-acetamid,
cis-4-[3-[4-(1,1-Dimethylpropyl)-phenyl-2-methylpropyl]-2,6-dimethyl-morpholinhydrochlorid,
1-(3,5 Dichlorphenyl)-3-(2-propenyl)-2,5-pyrrolidindion,
1-Methyl-5-nonyl-2-(phenylmethyl)-3-pyrrolidinol,
1-[[2-(4-Chlorphenyl)-3-phenyloxiranyl]-methyl]-1H-1,2,4-triazol,
Methantetrathiol-Natriumsalz,
2-(2,3,3-Triiod-2-propenyl)-2H-tetrazol,
N-[3-Chlor-4,5-bis(2-propinyloxy)-phenyl]-N'-methoxy-methanimidamid,
α-(5-Methyl-1,3-dioxan-5-yl)-β-[[4-(trifluormethyl)-phenyl]-methylen]-1H-1,2,4-triazol-1-ethanol,
1-(2-Methyl-1-naphthalenyl)-1H-pyrrol-2,5-dion,
N-(2,6-Dimethylphenyl)-2-methoxy-N-(tetrahydro-2-oxo-3-furanyl)-acetamid,
3,4-Dichlor-1-[4-(difluormethoxy)-phenyl]-1H-pyrrol-2,5-dion,
N-[2,2,2-Trichlor-1-[(chloracetyl)-amino)-ethyl]-benzamid,
N-Formyl-N-hydroxy-DL-alanin-Natriumsalz,
N-(4-Cyclohexylphenyl)-1,4,5,6-tetrahydro-2-pyrimidinamin,
4-Methyl-tetrazolo[1,5-a]quinazolin-5(4H)-on,
2-Chlor-N-(2,6-dimethylphenyl)-N-(isothiocyanatomethyl)-acetamid,
Ethyl-[(4-chlorphenyl)-azo]-cyanoacetat,
N-(4-Hexylphenyl)-1,4,5,6-tetrahydro-2-pyrimidinamin,
N-(2-Chlor-4-nitrophenyl)-4-methyl-3-nitro-benzolsulfonamid,
Methyl-N-(chloracetyl)-N-(2,6-dimethylphenyl)-DL-alaninat,
3-[2-(4-Chlorphenyl)-5-ethoxy-3-isoxazolidinyl]-pyridin,
2-[(1-Methylethyl)sulfonyl]-5-(trichlormethyl)-1,3,4-thiadiazol,
spiro[2H]-1-Benzopyran-2,1'(3'H)-isobenzofuran]-3'-on,
Methyl-N-(2,6-dimethylphenyl)-N-(5-isoxazolylcarbonyl)-DL-alaninat,
Kaliumhydrogencarbonat,
1-[[2-(2,4-Dichlorphenyl)-1,3-dioxolan-2-yl]-methyl]-1H-imidazol,
1-[(Diiodmethyl)-sulfonyl]-4-methyl-benzol,
2-Brom-2-(brommethyl)-pentandinitril,
2-[[6-Deoxy-4-O-(4-O-methyl-β-D-glycopyranosyl)-α-D-glucopyranosyl]-amino]-4-methoxy-1H-pyrrolo[2,3-d]pyrimidin-5-carbonitril,
Methyl-1-(2,3-dihydro-2,2-dimethyl-1H-inden-1-yl)-1H-imidazol-5-carboxylat,
2-Chlor-N-(2,3-dihydro-1,1,3-trimethyl-1H-inden-4-yl)-3-pyridincarboxamid,
O,O-Diethyl-[2-(dipropylamino)-2-oxoethyl]-ethylphosphoramidothioat,
α-(2,4-Dichlorphenyl)-β-fluor-β-propyl-1H-1,2,4-triazol-1-ethanol,
3-(1,1-Dimethylpropyl-1-oxo-1H-inden-2-carbonitril,
2,6-Dichlor-5-(methylthior-4-pyrimidinyl-thiocyanat,
S-Methyl-1,2,3-benzothiadiazol-7-carbothioat,
N-(6-Methoxy)-3-pyridinyl)-cyclopropancarboxamid,
3,5-Dichlor-N-[cyan-[(1-methyl-2-propynyl)-oxy]-methyl]-benzamid,
4-Chlor-2-cyan-N,N-dimethyl-5-(4-methylphenyl)-1H-imidazol-1-sulfonamid,
8-(1,1-Dimethylethyl)-N-ethyl-N-propyl-1,4-dioxaspiro[4.5]decan-2-methanamin,
2,2-Dichlor-N-[1-(4-chlorphenyl)-ethyl]-1-ethyl-3-methyl-cyclopropancarboxamid,
N-(2,3-Dichlor-4-hydroxyphenyl)-1 -methyl-cyclohexancarboxamid.

### Bakterizide:

Bronopol, Dichlorophen, Nitrapyrin, Nickel Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxim, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos , Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin, Lambda-cyhalothrin, Lufenuron,
Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyridaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Die zum Schutz technischer Materialien verwendeten Mittel enthalten die Wirkstoffe im allgemeinen in einer Menge von 1 bis 95%, bevorzugt von 10 bis 75 %.

Die Anwendungskonzentrationen der erfindungsgemäßen Wirkstoffe richten sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gewichts-%, vorzugsweise von 0,05 bis 1,0 Gewichts-% bezogen auf das zu schützende Material.

Die Wirksamkeit und das Wirkungsspektrum der erfindungsgemäß im Materialschutz zu verwendenden Wirkstoffe hzw der daraus herstellbaren Mittel, Konzentrate oder ganz allgemein Formulierungen kann erhöht werden, wenn gegebenenfalls weitere antimikrobiell wirksame Verbindungen, Fungizide, Bakterizide, Herbizide, Insektizide oder andere Wirkstoffe zur Vergrößerung des Wirkungsspektrums oder Erzielung besonderer Effekte wie z.B. dem zusätzlichen Schutz vor Insekten zugesetzt werden. Diese Mischungen können ein breiteres Wirkungsspektrum besitzen als die erfindungsgemäßen Verbindungen.

Die Herstellung und die Verwendung der erfindungsgemäßen Stoffe gehen aus den folgenden Beispielen hervor.

### Herstellungsbeispiele

### Beispiel 1

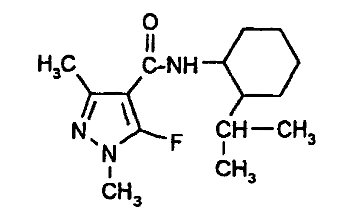

In ein Gemisch aus 2,1 g (0,015 Mol) 2-Isopropyl-cyclohexylamin, 1,5 g (0,015 Mol) Triethylamin und 40 ml Toluol wird bei Raumtemperatur unter Rühren eine Lösung von 2,6 g (0,015 Mol) 5-Fluor-1,3-dimethyl-pyrazol-4-carbonsäurechlorid in 10 ml Toluol eingetropft. Nach beendeter Zugabe wird das Reaktionsgemisch noch 2 Stunden bei Raumtemperatur gerührt und dann mit Wasser versetzt. Die organische Phase wird abgetrennt, über Natriumsulfat getrocknet und unter vermindertem Druck eingeengt. Der verbleibende Rückstand wird mit n-Hexan verrührt. Das dabei anfallende Festprodukt wird abgesaugt und getrocknet. Man erhält auf diese Weise 3,6 g (85,4 % der Theorie) an 5-Fluor-1,3-dimethyl-pyrazol-4-carbonsäure-(2-isopropyl)-cyclohexylamid.
Fp. 126°C

In analoger Weise werden die in der folgenden Tabelle aufgeführten 1,3-Dimethyl-5-fluor-pyrazol-4-carboxamide der Formel (I) hergestellt

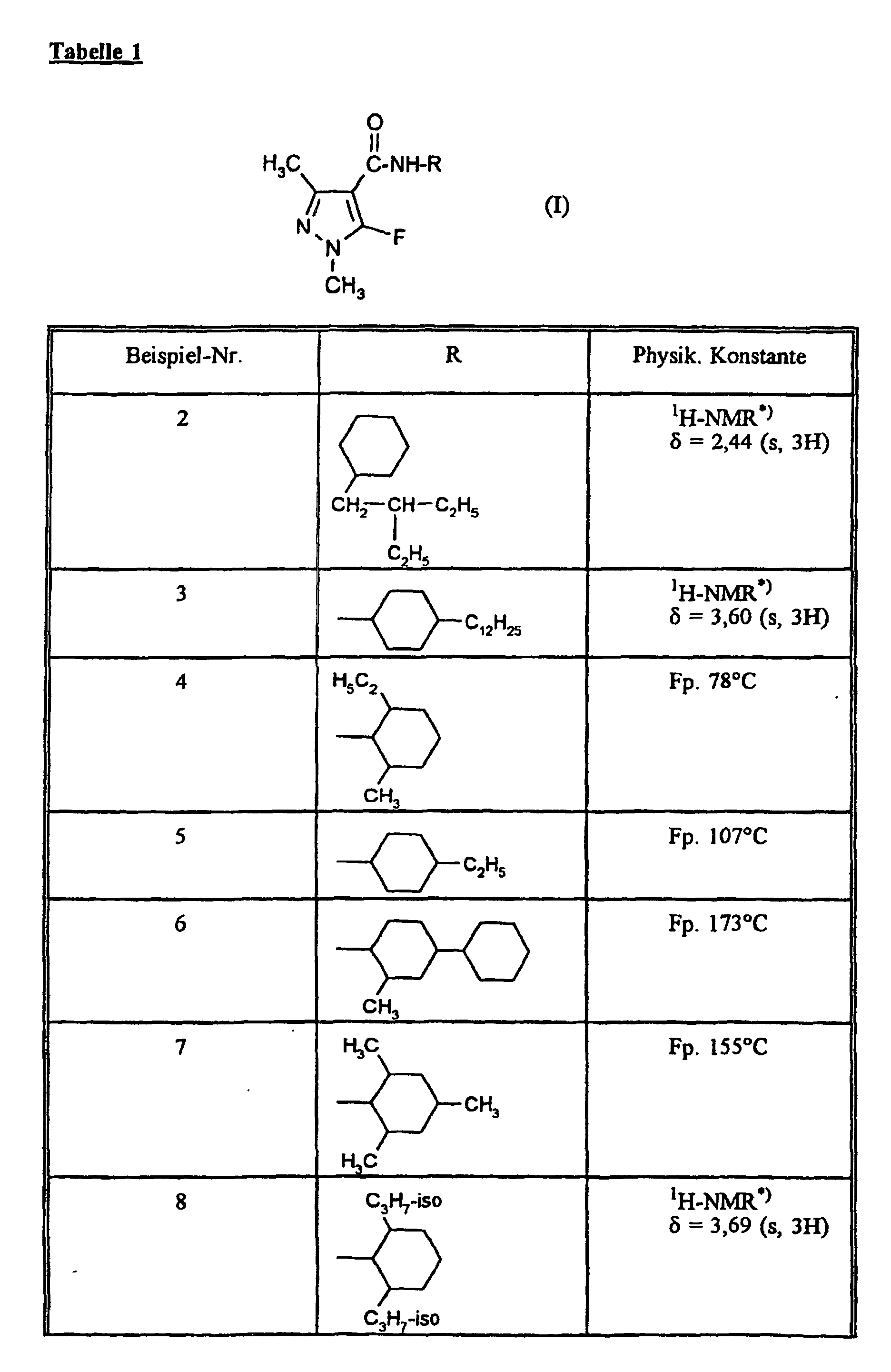

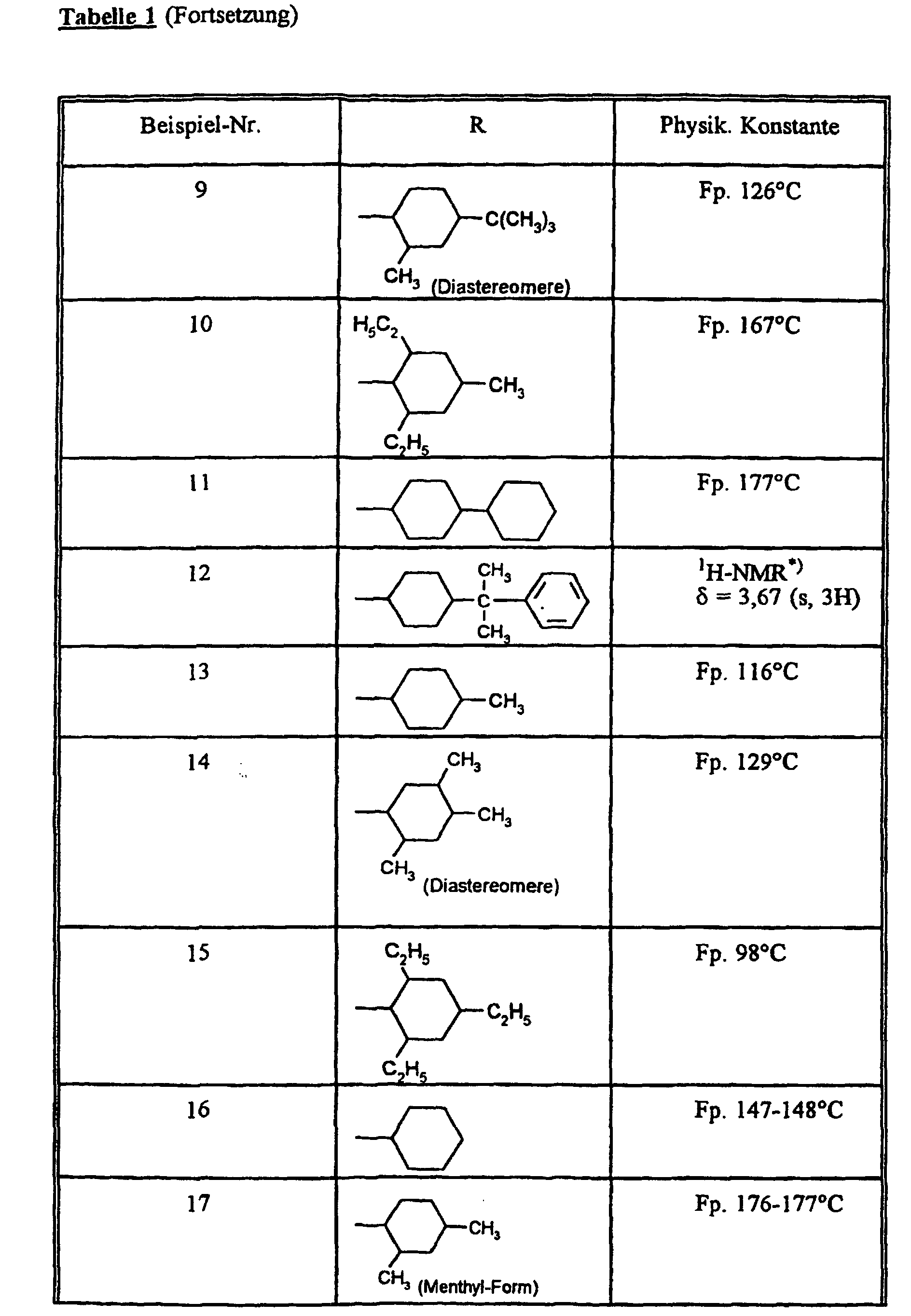

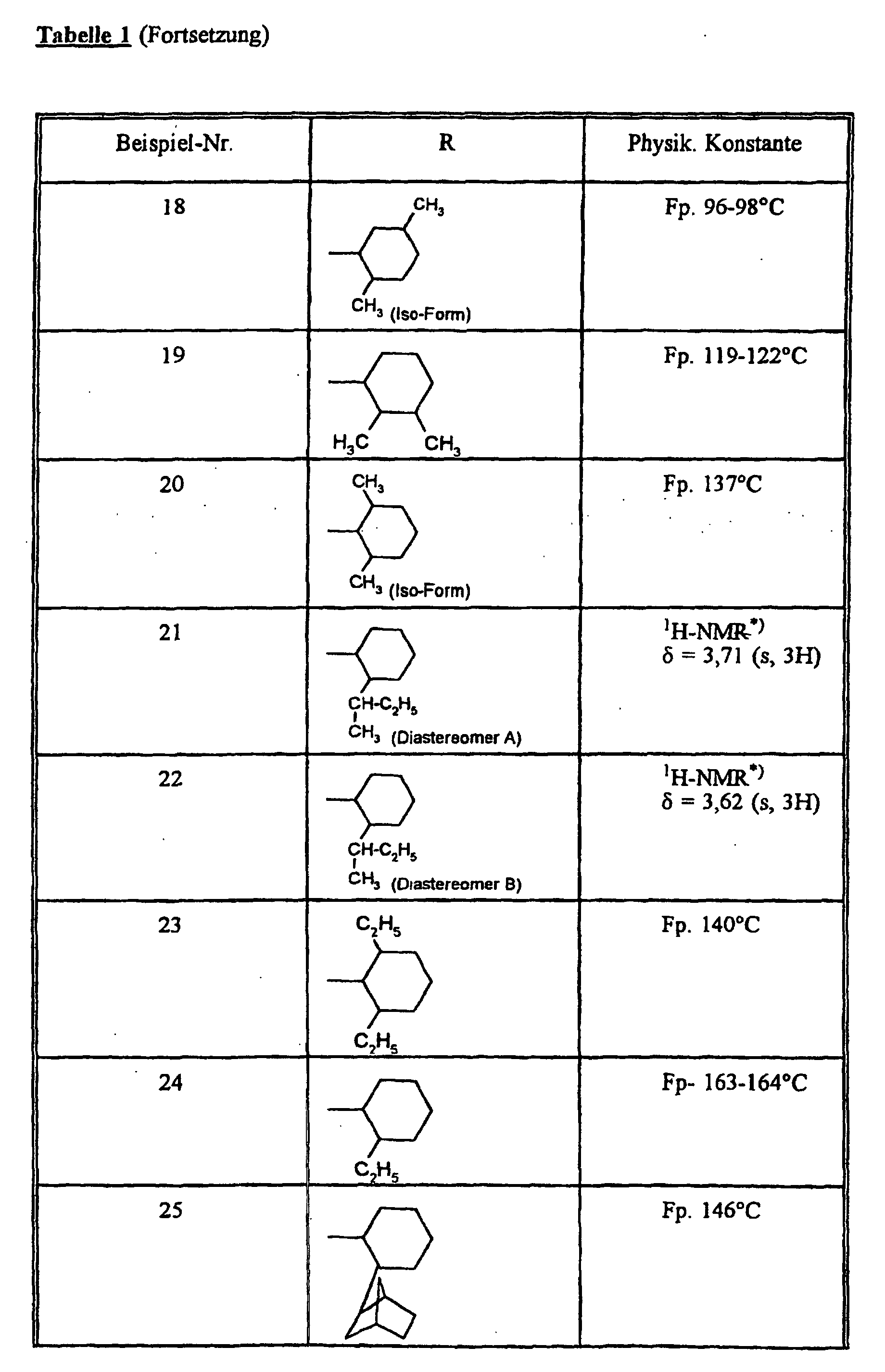

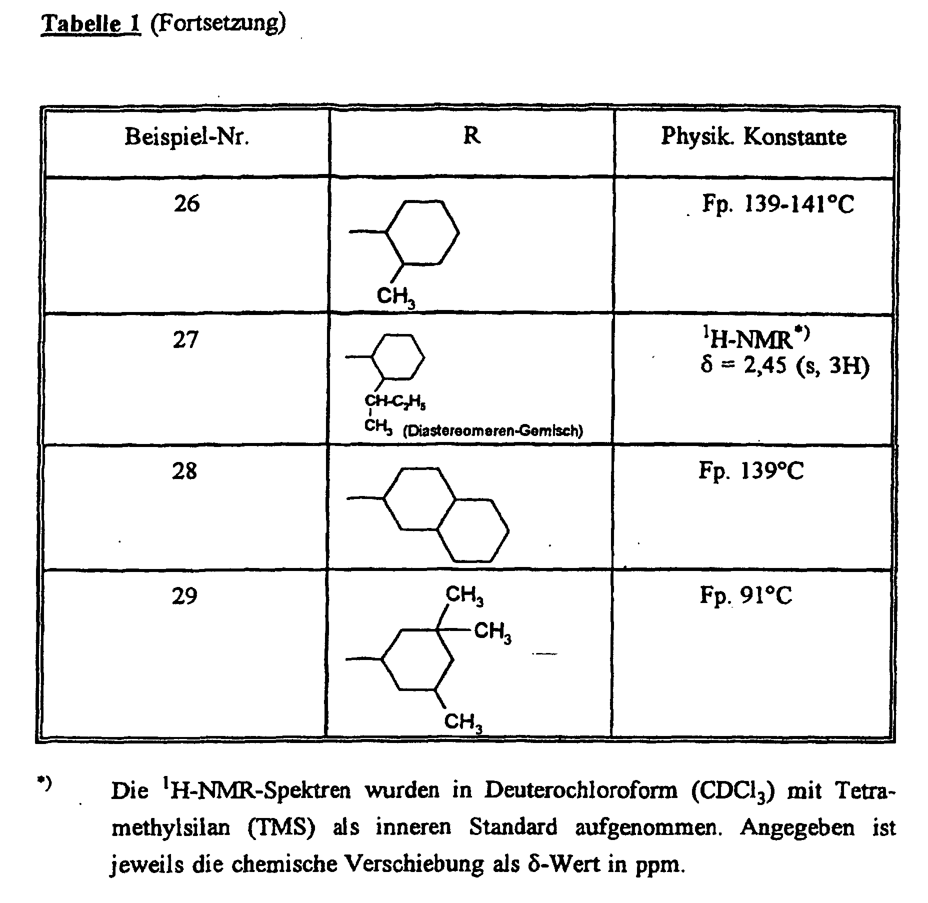

### Verwendungsbeispiele

### Beispiel A

### Erysiphe-Test (Gerste) / protektiv

| | |
|---|---|
| Lösungsmittel | 10 Gewichtsteile N-Methyl-pyrrolidon |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstgen.

7 Tage nach der Inokulation erfolgt die Auswertung. Dabei bedeutet 0 % ein Wirkungsgrad, der demjenigen der unbehandelten Kontrolle entspricht, während ein Wirkungsgrad von 100 % bedeutet, daß kein Befall beobachtet wird.

Wirkstoffe, Aufwandmengen und Versuchsergebnisse gehen aus der folgenden Tabelle hervor.

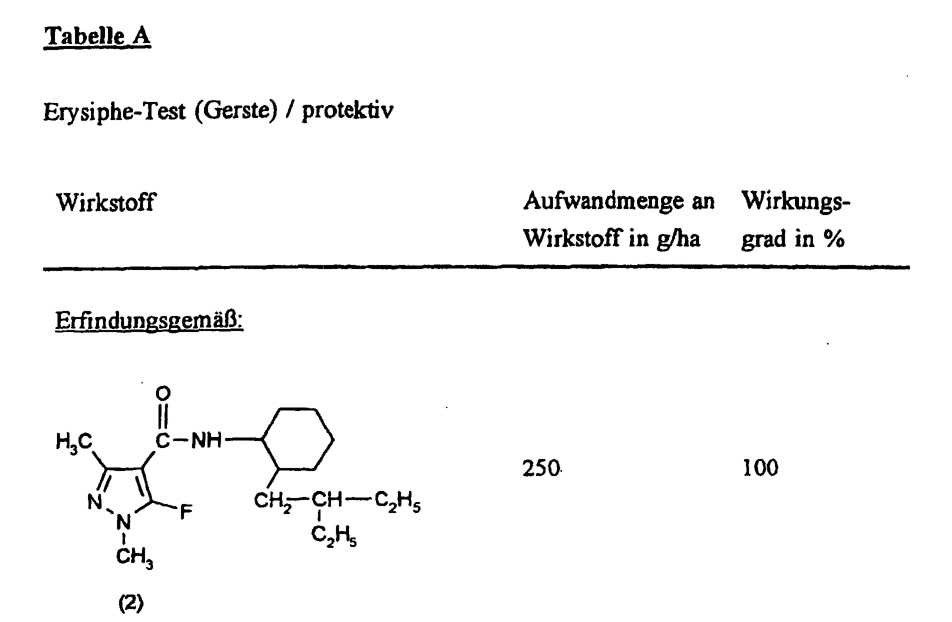

### Beispiel B

### Pyrenophora teres-Test (Gerste) / protektiv

| | |
|---|---|
| Lösungsmittel | 10 Gewichtsteile N-Methyl-pyrrolidon |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit einer Konidiensuspension von Pyrenophora teres besprüht.

Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt.

7 Tage nach der Inokulation erfolgt die Auswertung. Dabei bedeutet 0 % ein Wirkungsgrad, der demjenigen der unbehandelten Kontrolle entspricht, während ein Wirkungsgrad von 100 % bedeutet, daß kein Befall beobachtet wird.

Wirkstoffe, Aufwandmengen und Versuchsergebnisse gehen aus der folgenden Tabelle hervor.

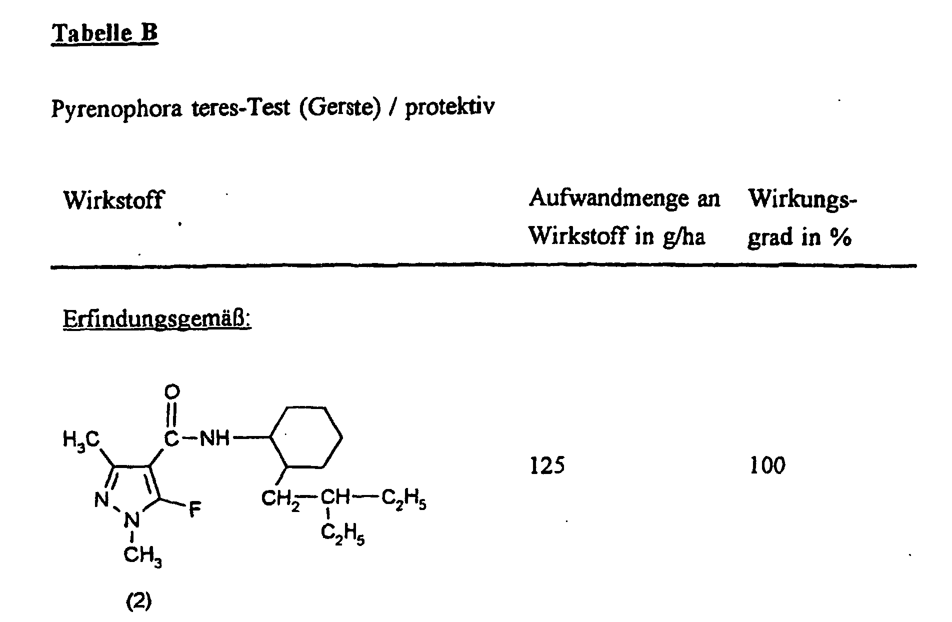

### Beispiel C

### Materialschutz-Test

Zum Nachweis der Wirksamkeit gegen Pilze werden die minimalen Hemmkonzentrationen (MKH-Werte) von erfindungsgemäßen Verbindungen bestimmt:
Ein Agar, der unter Verwendungvon Malzextrakt hergestellt wird, wird mit erfindungsgemäßen Wirkstoffen in Konzentrationen von 0,1 mg/l bis 5.000 mg/l versetzt. Nach Erstarren des Agars erfolgt Kontamination mit Reinkulturen von Testorganismen. Nach zweiwöchiger Lagerung bei 28°C und 60 bis 70 % relativer Luftfeuchtigkeit wird die minimale Hemmkonzentration (MKH-Wert) bestimmt. Der MKH-Wert kennzeichnet die niedrigste Konzentration an Wirkstoff, bei der keinerlei Bewuchs durch die verwendete Mikrobenart erfolgt.
Wirkstoffe und Versuchsergebnisse gehen aus der folgenden Tabelle hervor.

**Tabelle C**

| Materialschutz-Test Minimale Hemmkonzentration (MKH-Wert) in mg/l | | |
|---|---|---|
| Testorganismus | Wirkstoff gemäß Beispiel Nr. | |
| | 16 | 19 |
| Chaetomium globosum | 100 | 100 |
| Aspergillus niger | 200 | 400 |

### Beispiel D

### Sphaerotheca-Test (Gurke) / protektiv

| | |
|---|---|
| Lösungsmittel | 47 Gewichtsteile Aceton |
| Emulgator | 3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von Sphaerotheca fuliginea inokuliert. Die Pflanzen werden dann bei ca. 23°C und einer relativen Luftfeuchtigkeit von ca. 70 % im Gewächshaus aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Wirkstoffe, Aufwandmengen und Versuchsergebnisse gehen aus der folgenden Tabelle hervor.

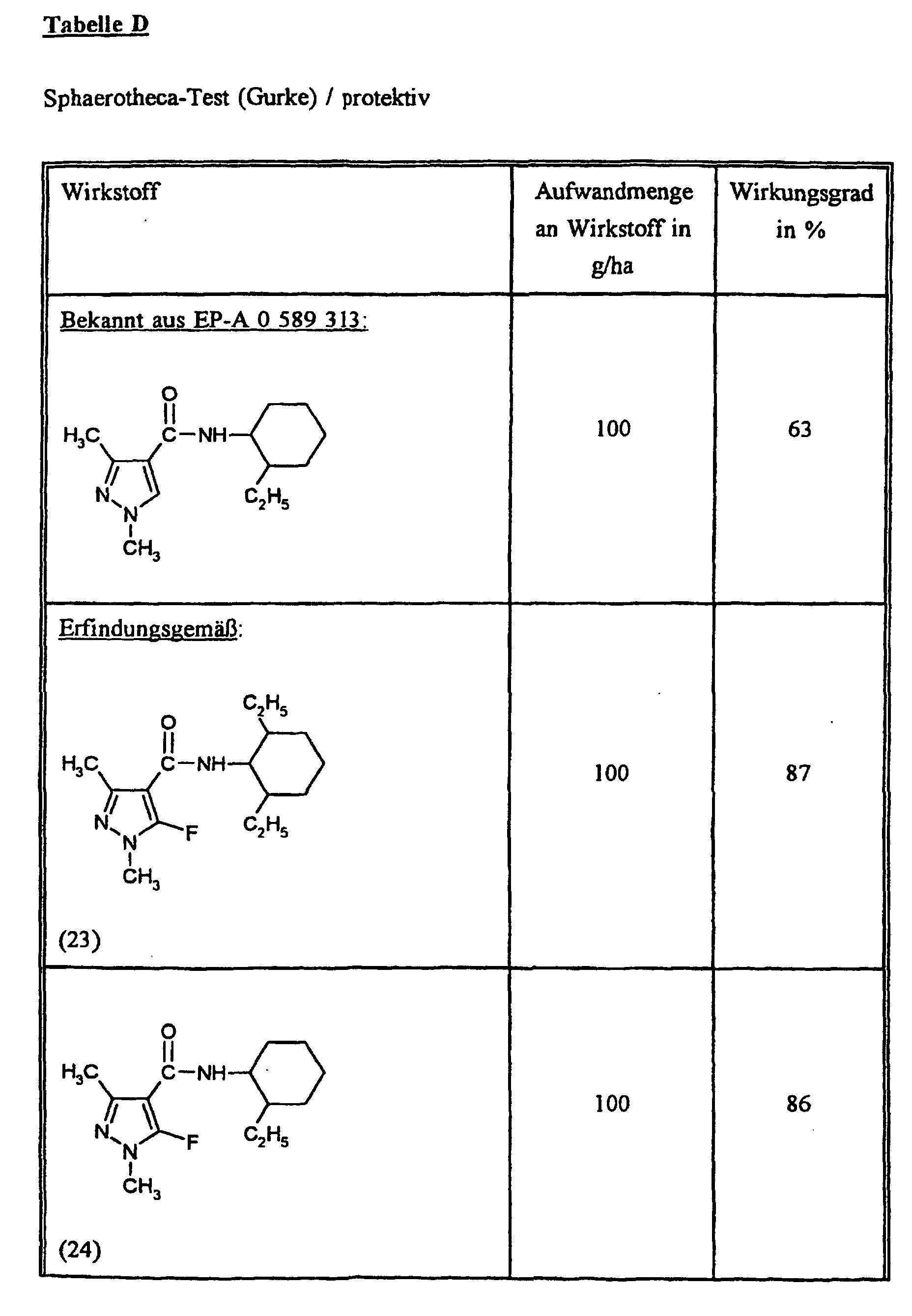

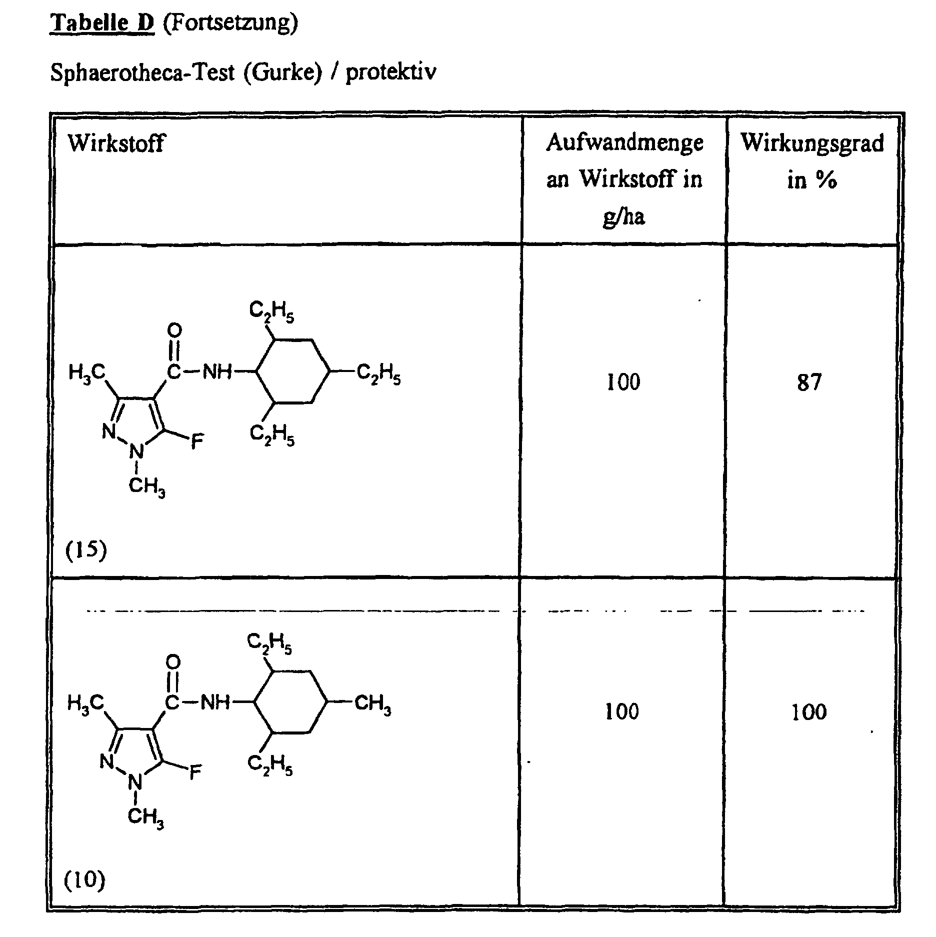

### Beispiel E

### Venturia-Test (Apfel) / protektiv

| | |
|---|---|
| Lösungsmittel | 47 Gewichtsteile Aceton |
| Emulgator | 3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Konidiensuspension des Apfelschorferregers Venturia inaequalis inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

12 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobactet wird.

Wirkstoffe, Aufwandmengen und Versuchsergebnisse gehen aus der folgenden Tabelle hervor.

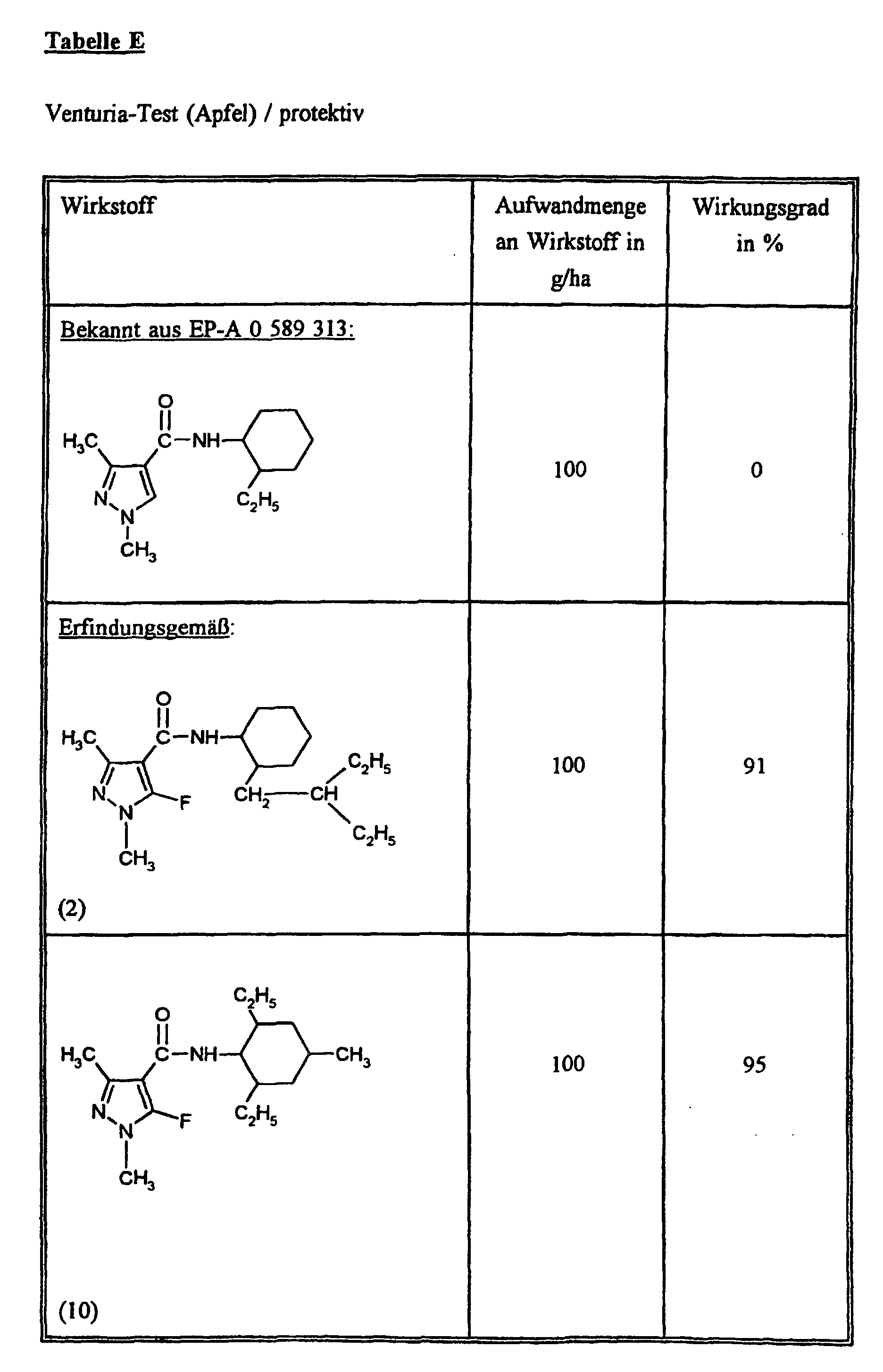

## Patentansprüche

1. 1,3-Dimethyl-5-fluor-pyrazol-4-carboxamide der Formel 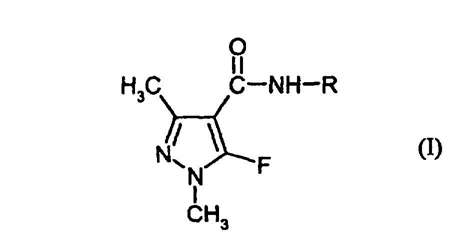 in welcher
R für eine Gruppierung der Formel 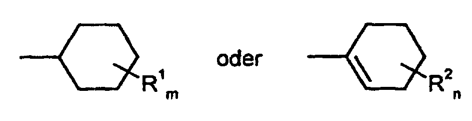 steht, worin
R¹ für geradkettiges oder verzweigtes Alkyl mit 1 bis 12 Kohlenstoffatomen, Cycloalkyl mit 3 bis 8 Kohlenstoffatomen, Bicycloalkyl mit 7 bis 12 Kohlenstoffatomen, gegebenenfalls einfach bis dreifach, gleichartig oder verschieden durch Halogen und/oder Alkyl mit 1 bis 4 Kohlenstoffatomen substituiertes Phenyl oder für gegebenenfalls einfach bis dreifach, gleichartig oder verschieden durch Halogen und/oder Alkyl mit 1 bis 4 Kohlenstoffatomen substituiertes Phenylalkyl mit 1 bis 4 Kohlenstoffatomen im Alkylteil steht,
m für die Zahlen 0, 1, 2 oder 3 steht,
oder
zwei vicinale R¹-Reste gemeinsam für eine Alkylenkette mit 3 oder 4 Kohlenstoffatomen stehen,
R² für geradkettiges oder verzweigtes Alkyl mit 1 bis 12 Kohlenstoffatomen, Cycloalkyl mit 3 bis 8 Kohlenstoffatomen, Bicycloalkyl mit 7 bis 12 Kohlenstoffatomen, gegebenenfalls einfach bis dreifach, gleichartig oder verschieden durch Halogen und/oder Alkyl mit 1 bis 4 Kohlenstoffatomen substituiertes Phenyl oder für gegebenenfalls einfach bis dreifach, gleichartig oder verschieden durch Halogen und/oder Alkyl mit 1 bis 4 Kohlenstoffatomen substituiertes Phenylalkyl mit 1 bis 4 Kohlenstoffatomen im Alkylteil steht und
n für die Zahlen 0, 1, 2 oder 3 steht,
oder
zwei vicinale R²-Reste gemeinsam für eine Alkylenkette mit 3 oder 4 Kohlenstoffatomen stehen.

2. Verfahren zur Herstellung von 1,3-Dimethyl-5-fluor-pyrazol-4-carboxamiden der Formel (I) gemäß Anspruch 1, **dadurch gekennzeichnet, dass** man 1,3-Dimethyl-5-fluor-pyrazol-4-carbonsäure-halogenide der Formel 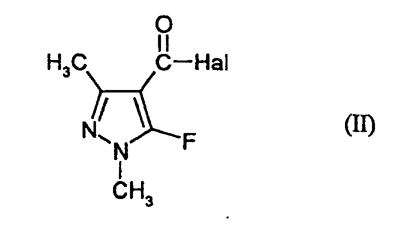 in welcher
Hal für Fluor, Chlor oder Brom steht,
mit Aminen der Formel
in welcher
R die in Anspruch 1 angegebenen Bedeutungen hat,
gegebenenfalls in Gegenwart eines Säurebindemittels und gegebenenfalls in Gegenwart eines Verdünnungsmittels umsetzt.

3. Mikrobizide Mittel, **gekennzeichnet durch** einen Gehalt an mindestens einem 1,3-Dimethyl-5-fluor-pyrazol-4-carboxamid der Formel (I) gemäß Anspruch 1.

4. Verwendung von 1,3-Dimethyl-5-fluor-pyrazol-4-carboxamiden der Formel (I) gemäß Anspruch 1 als Mikrobizide im Pflanzenschutz und im Materialschutz.

5. Verfahren zur Bekämpfung von unerwünschten Mikroorganismen im Pflanzenschutz und im Materialschutz, **dadurch gekennzeichnet, dass** man 1,3-Dimethyl-5-fluor-pyrazol-4-carboxamide der Formel (I) gemäß Anspruch 1 auf die Mikroorganismen und/oder deren Lebensraum ausbringt.

6. Verfahren zur Herstellung von mikrobiziden Mitteln, **dadurch gekennzeichnet, dass** man 1,3-Dimethyl-5-fluor-pyrazol-4-carboxamide der Formel (I) gemäß Anspruch 1 mit Streckmitteln und/oder oberflächenaktiven Stoffen vermischt.

## Claims

1. 1,3-Dimethyl-5-fluoro-pyrazol-4-carboxamides of the formula 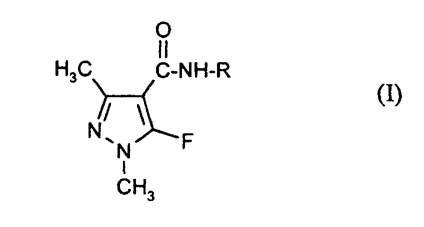 in which
R represents a grouping of the formula 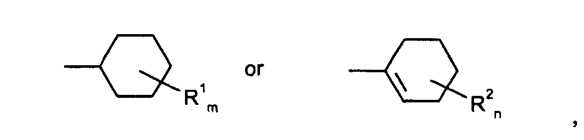 in which
R¹ represents a straight-chain or branched alkyl having 1 to 12 carbon atoms, cycloalkyl having 3 to 8 carbon atoms, bicycloalkyl having 7 to 12 carbon atoms, phenyl which is optionally mono- to trisubstituted by identical or different substituents selected from the group consisting of halogen and alkyl having 1 to 4 carbon atoms or represents phenylalkyl having 1 to 4 carbon atoms in the alkyl moiety which is optionally mono- to trisubstituted by identical or different substituents selected from the group consisting of halogen and alkyl having 1 to 4 carbon atoms,
m represents the numbers 0, 1, 2 or 3,
or
two vicinal R' radicals together represent an alkylene chain having 3 or 4 carbon atoms,
R² represents a straight-chain or branched alkyl having 1 to 12 carbon atoms, cycloalkyl having 3 to 8 carbon atoms, bicycloalkyl having 7 to 12 carbon atoms, phenyl which is optionally mono- to trisubstituted by identical or different substituents selected from the group consisting of halogen and alkyl having 1 to 4 carbon atoms or represents phenylalkyl having 1 to 4 carbon atoms in the alkyl moiety which is optionally mono- to trisubstituted by identical or different substituents selected from the group consisting of halogen and alkyl having 1 to 4 carbon atoms and
n represents the numbers 0, 1, 2 or 3,
or
two vicinal R² radicals together represent an alkylene chain having 3 or 4 carbon atoms.

2. Process for preparing 1,3-dimethyl-5-fluoro-pyrazol-4-carboxamides of the formula (I) according to Claim 1, **characterized in that** 1,3-dimethyl-5-fluoro-pyrazol-4-carbonyl halides of the formula 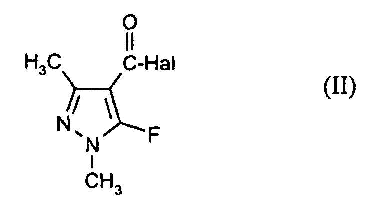 in which
Hal represents fluorine, chlorine or bromine,
are reacted with amines of the formula
H₂N-R (III)
in which
R is as defined in Claim 1,
if appropriate in the presence of an acid binder and if appropriate in the presence of a diluent.

3. Microbicidal compositions, **characterized in that** they contain at least one 1,3-dimethyl-5-fluoro-pyrazol-4-carboxamide of the formula (I) according to Claim 1.

4. Use of 1,3-dimethyl-5-fluoro-pyrazol-4-carboxamides of the formula (I) according to Claim 1 as microbicides in crop protection and in the protection of materials.

5. Method for controlling undesirable microorganisms in crop protection and in the protection of materials, **characterized in that** 1,3-dimethyl-5-fluoro-pyrazol-4-carboxamides of the formula (I) according to Claim 1 are applied to the microorganisms and/or their habitat.

6. Process for preparing microbicidal compositions, **characterized in that** 1,3-dimethyl-5-fluoro-pyrazol-4-carboxamides of the formula (I) according to Claim 1 are mixed with extenders and/or surfactants.

## Revendications

1. 1,3-diméthyl-5-fluoropyrazole-4-carboxamide de formule 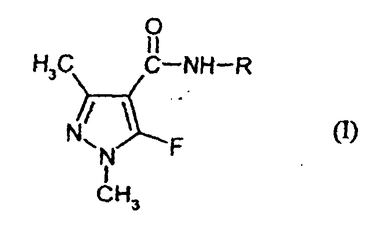 dans laquelle
R représente un groupement de formule 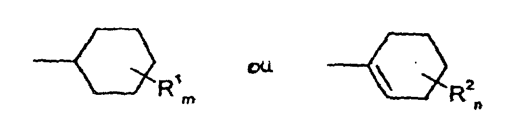
dans laquelle
R¹ est un reste alkyle linéaire ou ramifié ayant 1 à 12 atomes de carbone, un reste cycloalkyle ayant 3 à 8 atomes de carbone, un reste bicycloalkyle ayant 7 à 12 atomes de carbone, un reste phényle éventuellement substitué une à trois fois, identiques ou différentes, par un halogène et/ou un radical alkyle ayant 1 à 4 atomes de carbone, ou un reste phénylalkyle ayant 1 à 4 atomes de carbone dans la partie alkyle, éventuellement substitué une à trois fois, identiques ou différentes par un halogène et/ou un radical alkyle ayant 1 à 4 atomes de carbone,
m représente les nombres 0, 1, 2 ou 3,
ou bien '
deux restes R¹ vicinaux forment ensemble une chaîne alkylénique ayant 3 ou 4 atomes de carbone,
R² représente un reste alkyle linéaire ou ramifié ayant 1 à 12 atomes de carbone, un reste cycloalkyle ayant 3 à 8 atomes de carbone, un reste bicycloalkyle ayant 7 à 12 atomes de carbone, un reste phényle éventuellement substitué une à trois fois, identiques ou différentes, par un halogène et/ou un radical alkyle ayant 1 à 4 atomes de carbone, ou un reste phénylalkyle ayant 1 à 4' atomes de carbone dans la partie alkyle, éventuellement substitué une à trois fois, identiques ou différentes, par un halogène et/ou un radical alkyle ayant 1 à 4 atomes de carbone, et
n représente les nombres 0, 1, 2 ou 3,
ou bien
deux restes R² vicinaux forment ensemble une chaîne alkylénique ayant 3 ou 4 atomes de carbone.

2. Procédé de production de 1,3-diméthyl-5-fluoropyrazole-4-carboxamide de formule (I) suivant la revendication 1, **caractérisé en ce qu'**on fait réagir des halogénures d'acide 1,3-diméthyl-5-fluoropyrazole-4-carboxylique de formule 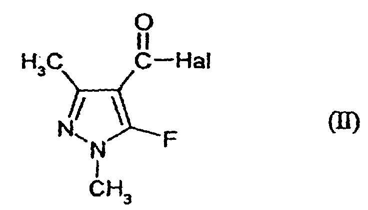 dans laquelle
Hal représente le fluor, le chlore ou le brome,
avec des amines de formule
H₂N-R (III)
dans laquelle
R a les définitions indiquées dans la revendication 1, éventuellement en présence d'un accepteur d'acide et, le cas échéant, en présence d'un diluant.

3. Compositions microbicides, **caractérisées par** une teneur en au moins un 1,3-diméthyl-5-fluoropyrazole-4-carboxamide de formule (I) suivant la revendication 1.

4. Utilisation de 1,3-diméthyl-5-fluoropyrazole-4-carboxamides de formule (I) suivant la revendication 1 comme microbicides dans la protection des plantes et dans la protection des matériaux.

5. Procédé de lutte contre des microorganismes indésirables dans la protection des plantes et dans la protection des matériaux, **caractérisé en ce qu'**on épand des 1,3-diméthyl-5-fluoropyrazole-4-carboxamides de formule (I) suivant la revendication 1 sur les microorganismes et/ou sur leur milieu.

6. Procédé de préparation de compositions microbicides, **caractérisé en ce qu'**on mélange des 1,3-diméthyl-5-fluoropyrazole-4-carboxamides de formule (I) suivant la revendication 1 avec des diluants et/ou des agents tensioactifs.
